Europäisches Patentamt

European Patent Office

Office européen des brevets

(11) Veröffentlichungsnummer: **0 009 754**
**B1**

(12) **EUROPÄISCHE PATENTSCHRIFT**

(45) Veröffentlichungstag der Patentschrift: **08.02.84**

(21) Anmeldenummer: **79103632.0**

(22) Anmeldetag: **25.09.79**

(51) Int. Cl.³: **C 08 F 265/06,**
**A 61 L 15/06**

(54) **Selbsthärtende Masse auf der Basis von Polymethacrylaten, Verfahren zu ihrer Herstellung und ihre Verwendung.**

(30) Priorität: **02.10.78 DE 2842839**

(43) Veröffentlichungstag der Anmeldung:
**16.04.80 Patentblatt 80/8**

(45) Bekanntmachung des Hinweises auf die Patenterteilung:
**08.02.84 Patentblatt 84/6**

(84) Benannte Vertragsstaaten:
**AT BE CH FR GB IT NL SE**

(56) Entgegenhaltungen:
**DE - A - 1 544 924**
**DE - A - 2 739 282**
**FR - A - 1 381 751**
**FR - E - 69 263**
**US - A - 4 104 333**

(73) Patentinhaber: **NATEC Institut für
naturwissenschaftlich- technische Dienste GmbH
Behringstrasse 154
D-2000 Hamburg 50 (DE)**

(72) Erfinder: **Seidel, Hartmut, Dr.
Hochrad 19
D-2000 Hamburg 52 (DE)**
Erfinder: **Polzhofer, Kurt, Dr.
Bellmannstrasse 8
D-2000 Hamburg 52 (DE)**

(74) Vertreter: **UEXKÜLL & STOLBERG Patentanwälte
Beselerstrasse 4
D-2000 Hamburg 52 (DE)**

Selbsthärtende Masse auf der Basis von Polymethacrylaten, Verfahren zu ihrer Herstellung und ihre Verwendung

Vorliegende Erfindung betrifft eine selbsthärtende Masse auf der Basis von Polymethacrylaten, die insbesondere in der Knochenchirurgie vorteilhaft verwendet werden kann, sowie ein Verfahren zu ihrer Herstellung.

Selbsthärtende Kunststoffe sind seit langem bekannt und werden auch seit geraumer Zeit in der operativen Orthopädie angewendet. An einen für eine Implantation vorgesehenen Werkstoff müssen ganz besondere Anforderungen gestellt werden, beispielsweise chemische und mechanische Stabilität auch unter der langzeitigen Einwirkung von Körperflüssigkeiten, biologische Verträglichkeit, Herstellbarkeit in jeder gewünschten Form, gute Sterilisierbarkeit usw. Gleichzeitig sollen nach Möglichkeit auch Wirtschaftlichkeit und Preiswürdigkeit bei der Anwendung solcher Stoffe gegeben sein. Es ist daher nicht erstaunlich, daß sich die Auswahl geeigneter Produkte von vornherein auf nur wenige Kunststofftypen beschränkte, von denen sich insbesondere die Polymethylmethacrylate in der chirurgischen Orthopädie haben behaupten können.

Polymethylmethacrylate (PMMA) sind harte, biegsame, farblose thermoplastische Kunststoffe, die auch starken mechanischen Beanspruchungen standhalten und sich wegen ihrer biologischen Verträglichkeit und guten Sterilisierbarket zur Herstellung elastischer Prothesen eignen. So sind z.B. unter den Handelsnamen Paladon, Propalat und Palacos PMMA-Produkte als Zahnprothesenkunststoff bzw. als Knochenzement für die chirurgische Orthopädie seit langem bekannt. Im Prinzip handelt es sich hierbei um pulverförmige Polymere, die mit der entsprechenden monomeren Flüssigkeit (Methylmethacrylat) zu einem Teig angerührt werden, der je nach Art der weiteren Zusätze, insbesondere von Polymerisationskatalysatoren, bei Zimmertemperatur oder bei erhöhter Temperatur innerhalb kurzer Zeit zu einer sehr festen, homogenen Masse erstarrt.

Obwohl man diese PMMA-Produkte sei geraumer Zeit in der Knochenchirurgie einsetzt, ist ihre Anwendung durchaus nicht problemlos. Während ein geschickter Operateur beim Anrühren und Einsetzen der PMMA-Knochenzemente gewisse Probleme, wie die während der Polymerisation der Knochenzemente nicht zu vermeidende Wärmeentwicklung oder die Wirkung der eingesetzten MMA-Monomeren auf den Kreislauf und die Herztätigkeit des Patienten, in der Regel durch geeignete Maßnahmen auf ein für den Patienten annehmbares Maß reduzieren kann, entziehen sich insbesondere die Vorgänge, die sich im Knochenzement und an der Grenze von Knochenzement und Prothese, insbesondere aber an der Knochenzement-Knochen-Grenze nach der Implantation vollziehen, weitgehend den Steuerungsmöglichkeiten des Operateurs. So läßt sich ein deutliches Schrumpfen der bekannten, bisher eingesetzten PMMA-Polymeren beim vollständigen Aushärten nicht vermeiden, wobei allein die Schrumpfung des Monomeren während der Polymerisation nach Loskaek und Fox, J. Amer. Chem. Soc. 75, 3544 (1953), bereits etwa 23% beträgt. Dieses Schrumpfen kann die feste Verankerung der Prothese in dem Knochenzement sowie die Verhältnisse an der Knochenzement-Knochen-Grenze nachhaltig negativ beeinflussen und wird als Ursache für zahlreiche Mißerfolge angesehen, die mit zunehmendem Abstand vom Zeitpunkt der Implantation der Prothesen immer häufiger auftreten.

Es ist daher bereits vorgeschlagen worden, die bekannten Knochenzemente durch Einarbeiten von monomerem 2-Hydroxyethylmethacrylat (HEMA) als Teil der flüssigen Monomerphase, die sonst aus monomerem Methylmethacrylat (MMA) besteht, zu modifizieren, wodurch die Wasseraufnahme des Zements in vivo gesteigert werden soll und möglicherweise auch das mechanische Ineinandergreifen im schwammig-porigen Knochen erhöht wird, siehe die Zusammenfassung eines Vortrags von G. P. Pearson veröffentlicht in den Tagungsunterlagen der 3. Konferenz "Mechanical Properties of Biomaterials", Universität Keele (GB), 13./15. Sept. 1978.

Aus DE—OS 2 739 782 ist eine Klebstoffmasse bekannt, mit der eine feste und dauernde Haftung an Knochen, Zahnbein und Zahnschmelz bewirkt werden soll. Diese bekannte Klebemasse erhält man ausschließlich aus einer flüssigen Monomerphase, für die als besonders geeignete Monomere u. a. Methylacrylat oder Methylmethacrylate sowie Hydroxyethylacrylat oder Hydroyethylmethacrylat angegeben werden. Diese flüssige Monomerphasea muß mit einem ternären Peroxid/Amin/Sulfinat-Härtungssystem ausgehärtet werden. Zwar soll die Härtungszeit nicht mehr als 10 Minuten betragen, aber die flüssige Konsistenz und vor allem der hohe Anteil an monomeren Acrylaten bzw. Methacrylaten zu Beginn und während des Aushärtens machen diese bekannte Mischung ungeeignet für eine praktische Anwendung in der Knochenchirurgie, wo in der Mehrzahl der Fälle die Anwendung direkt am Patienten erfolgen muß. Wegen der mehr oder weniger starken Giftigkeit der monomeren Acrylate bzw. Methacrylate ist es nämlich wesentlich, daß die Monomeren nach dem Einfüllen der Masse in den Knochenhohlraum und nach dem Aushärten nur noch in einer möglichst geringen Menge vorliegen, damit sie die Kreislauf- und Herztätigkeit des Patienten nicht unzumutbar stark belasten. Diese wesentlichen Voraussetzungen für eine physiologisch unbedenkliche Anwendung eines Knochenzementes werden durch die aus der DE—PS 2 739 282 bekannten Klebemassen offensichtlich nicht erfüllt.

In ähnlicher Weise wird in der DE—OS 2 420 351 ein Zahnfüllmaterial beschrieben, das aus einer Komponenten A im wesentlichen aus ein oder mehreren hydrophilen und hydrophoben Monomeren, wie 2-Hydroxyethylacrylat bzw. -methacrylat, ggf. im Gemisch mit monomeren Acrylaten und Methacrylaten, und aus einer Komponenten B aus einem Polymerisationskatalysator oder Beschleuniger, ggf. mit einem aktiven Füllstoff und einem Lösungsmittel, besteht. Damit das hydrophile Füllmaterial auch in die engen Hohlräume des Wurzelkanals eindringen und die Ritzen ausfüllen kann, muß das Material in fließbarem Zustand vorliegen und soll erst nach dem Eindringen in den Wurzelkanal und in die Ritzen aushärten und dadurch den Wurzelkanal versiegeln. Ein solches Vorgehen ist aus den oben genannten Gründen bei der Anwendung in dern Knochenchirurgie wegen der zu starken Belastung des Patienten nicht zumutbar.

Schließlich sind aus DE—PS 1 217 063, DE—OS 1 544 919 und DE—OS 1 544 924 Materialien zur Herstellung von Zahnprothesen bzw. von zahnärztlichem Reparaturmaterial auf der Basis von Polyacrylaten bzw. Polymethacrylaten bekannt, die nach dem "Pulver-Flüssigkeits-Verfahren" durch Polymerisation von monomerem Methylacrylat und/oder Methylmethacrylat und einem Zusatz von Acrylaten und Methacrylaten mit alkoholischen OH-Gruppen in Gegenwart von pulverförmigen Polymethylmethacrylat hergestellt werden. Da die Herstellung bzw. Reparatur von Zahnprothesen in der Regel außerhalb des menschlichen Körpers vorgenommen wird, kann die Aushärtung der bekannten Prothesenmaterialien bei Temperaturen von 100°C im Wasserbad erfolgen. Auch beim Aushärten des in der DE—OS 1 544 924 beschriebenen zahnärztlichen Reparaturmaterials tritt ein kräftiger Temperaturstoß auf, bei dem die Temperaturen in wenigen Minuten bis nahe 100°C ansteigen. Da solche Temperaturen weit über der Koagulationstemperatur der Eiweiße liegen, lassen sich diese bekannten Verfahren nicht in der Knochenchirurgie anwenden, wo der Knochenzement z.B. zur Verankerung einer Prothese in einen lebenden Knochen eingebracht werden muß.

Der vorliegenden Erfindung liegt die Aufgabe zugrunde, eine selbsthärtende Masse zu schaffen, die die vorgenannten Nachteile der bekannten Knochenzemente nicht zeigt und unabhängig von der Geschicklichkeit des Operateurs eine sichere Handhabung und Verträglichkeit gewährleistet und sich in einfacher Weise verarbeiten läßt. Diese Masse soll als Knochenzement in der Chirurgie und Orthopädie einsetzbar sein und sich bei und nach der Implantation günstig verhalten und insbesondere eine feste Verankerung sowohl mit dem Knochen als auch mit dem einzementierten Prothesenschaft ermöglichen.

Erfindungsgemäß wird diese Aufgabe durch eine selbsthärtende Masse auf Basis von Polymethacrylaten gelöst, die aus einem Gemisch aus einer feinpulvrigen, festen Polymerphase aus Polymethylmethacrylat und einer flüssigen Monomerphase aus Methylmethacrylat, ggf. zusammen mit 2-Hydroxyethylmethacrylat unter Zumischen weiterer üblicher Additive, wie geeigneten Initiatoren, Aktivatoren, Röntgenkontrastmitteln und Inhibitoren, zu einer oder beiden Phasen und kräftigem Rühren des erhaltenen Gemisches bei Raumtemperatur, wobei man einen knetbaren, plastisch verformbaren Teig erhält, der nach etwa 2 Minuten auszuhärten beginnt und nach etwa 10 Minuten ausgehärtet ist, hergestellt worden ist. Die erfindungsgemäße selbsthärtende Masse ist dadurch gekennzeichnet, daß die feste Polymerphase feinpulvriges Poly-2-hydroxyethylmethacrylat enthält, wobei das Gewichtsverhältnis von Polymerphase zu Monomerphase im Bereich von 1,5 bis 3,3 : 1 liegt und der Anteil an Poly-2-hydroxyethylmethacrylat in dem ausgehärteten Produkt mindestens 2 Gew.% und höchstens 99 Gew.% beträgt.

Überraschenderweise wurde festgestellt, daß das Anteigen der erfindungsgemäß vorgesehenen festen Polymerphase mit dem entsprechenden Monomeren Methylmethacrylat (MMA), ggf. zusammen mit 2-Hydroxyethylmethacrylat (HEMA), rasch zu einem plastischen Produkt führt, das bereits nach 2 Minuten nicht mehr schmierig und klebrig ist, sondern sich ohne Rest von der Unterlage bzw. von den Händen ablöst und sich daher in der Verarbeitbarkeit und Handhabbarkeit vorteilhaft von bisher bekannten Knochenzementen unterscheidet, die in der Regel erste 4 Minuten nach dem Anteigen eine Konsistenz erreichen, die nicht mehr schmierig und klebrig ist. Außerdem bleibt die erfindungsgemäße Masse längere Zeit plastisch, beispielsweise ist sie noch nach 6 Minuten ohne weiteres verarbeitbar. Dies ist insbesondere im Hinblick auf die Verträglichkeit für den Patienten von wesentlicher Bedeutung, da durch die Möglichkeit, den Zeitpunkt des Einfüllens des Knochenzements in den Knochenhohlraum so weit hinauszuschieben, bis der Knochenzement zu einem wesentlichen Teil bereits ausgehärtet ist, Sofortkomplikationen durch größere Mengen noch vorhandener Monomerer weitgehend ausgeschlossen werden können, da zum Zeitpunkt des Einfüllens der Monomerengehalt im noch verarbeitbaren Knochenzement bereits stark abgesunken ist.

Besonders im Vergleich mit bekannten Knochenzementen, die unter ausschließlicher Verwendung von PMMA in der Polymerphase und von MMA und HEMA in der flüssigen Monomerphase hergestellt werden bringt die erfindungsgemäß vorgeschlagene Zusammensetzung der festen Polymerphase aus PHEMA und PMMA zusätzliche Vorteile nicht nur im Hinblick auf die Aushärtezeiten, sondern auch hinsichtlich der Wärmeentwicklung während der Aushärtung mit sich. Durch den erfindungsgemäßen Einsatz von PHEMA als Teil der festen Polymerphase wird der Aushärtungsvorgang des Gemisches nämlich verlangsamt und die gebildete Wärmemenge besser über den gesamten Aushärtungsvorgang verteilt, so daß sie durch das umgebende Medium leichter abgeführt werden kann. Dies hat in der Praxis bei der Anwendung in der Knochenchirurgie erhebliche Vorzüge, insbensondere auch im Hinblick auf die geringere Belastung des Patienten während und nach der Operation.

Ferner besitzt die erfindungsgemäße selbsthärtende Masse auch die für eine dauerhafte Verankerung der Prothese im Knochen sehr wichtige feste Haftung an der Knochengrenze sowie ein ausgesprochen gutes Quellvermögen, so daß die erfindungsgemäße selbsthärtende Masse die Hohlräume in Knochen vollständig ausfüllen und durch den Quelldruck eine feste Verankerung sowohl an der Zement-Knochen-Grenze als auch mit dem einzementierten Prothesenschaft bilden kann. Hierbei wurde überraschend festgestellt, daß das der festen Polymerphase zugemischte feinpulvrige PHEMA eine deutlich günstigere Wirkung in der ausgehärteten Masse aufweist als der Zusatz von HEMA zur Monomerphase, was vermutlich auf die feste, körnige Struktur an der Oberfläche der ausgehärteten, ggf. gequollenen Masse zurückzuführen ist, die, wie aus Aufnahmen mit dem Rasterelektronenmikroskop zu erkennen, wesentlich ausgeprägter ist als bei ausgehärteten Massen, die unter ausschließlicher Verwendung von der Monomerphase zugesetztem HEMA hergestellt worden sind.

Vorzugsweise liegt das Gewichtsverhältnis von Polymerphase zu Monomerphase im Bereich von 2,0 bis 3,0:1. Die Polymerphase liegt zweckmäßig in feinpulvriger Form vor, vorzugsweise mit einer durchschnittlichen Korngröße von 0,1 $\mu$m bis 0,5 mm.

Die weiter unten näher erläuterten verbesserten Eigenschaften der erfindungsgemäßen selbsthärtenden Massen werden durch die im Anspruch 1 angegebenen Kombinationen von PMMA, PHEMA, MMA und ggf. HEMA erzielt, wobei der Anteil an PHEMA in dem ausgehärteten Produkt mindestens 2 Gew.% und höchstens 99 Gew.% beträgt.

In einer besonders bevorzugten Ausführungsform der Erfindung besteht die Polymerphase aus einem Gemisch von PMMA und PHEMA im Gewichtsverhältnis von 3,3 bis 10:1 und die Monomerphase aus MMA.

Damit die Aushärtung in der gewünschten Weise bei Raumtemperatur erfolgen kann, müssen im Gemisch aus flüssiger und fester Phase geeignete Initiatoren und Aktivatoren vorhanden sein. Als Initiatoren können ein oder mehrere Hydroperoxide und/oder ein oder mehrere organische oder anorganische Peroxide und als Aktivator ein oder mehrere Alkyl-, Alkylaryl- und/oder Oxyalkylamine vorteilhaft eingesetzt werden, wobei in der Regel der Initiator der Polymerphase und der Aktivator der Monomerphase zugemischt sind.

Die einzusetzenden Mengen an Initiator und Aktivator sind an sich nicht kritisch, sollen aus wirtschaftlichen Gründen aber möglichst niedrig sein. Es hat sich herausgestellt, daß die Aushärtung in der gewünschten Qualität bei Raumtemperatur oder wenig darunter oder darüber durchgeführt werden kann, wenn die Polymerphase vorzugsweise etwa 0,4 bis 1,0 Gew.% des Initiators und die Monomerphase vorzugsweise etwa 0,5 bis 0,7 Gew.% des Aktivators enthält. Der bevorzugte Initiator ist Dibenzoylperoxid, der bevorzugte Aktivator N,N-Dimethyl-p-toluidin.

Beispiele für geeignete Hydroperoxide sind tert.-Butylhydroperoxid und Cumol-hydroperoxid, Beispiele für geeignete organische Peroxide sind Di-tert.-butylperoxid, Methyl-ethylketon-peroxid, Methylisobutylketon-peroxid, Diacetyl-peroxid, Dicapronyl-peroxid, Dibenzoyl-peroxid, ferner Peroxysäuren und deren Ester, z.B. Peroxyessigsäure und tert. Butyl-peracetat.

Beispiele für geeignete Aktivatoren sind neben dem bereits genannten N,N-Dimethyl-p-toluidin auch Trimethylamin, Triethanolamin, Dimethylanilin und Diethylanilin.

Außerdem können den erfindungsgemäßen Mischungen weitere übliche Additive wie Röntgenkontrastmittel und Inhibitoren, beipsielsweise Ascorbinsäure, Alkylphenole, z.B. 2,2'-Methylen-bis-4-methyl-6-tert.-butylphenol und Hydrochinone, zugemischt werden.

Die Erfindung betrifft ferner ein Verfahren zur Herstellung der erfindungsgemäßen selbsthärtenden Masse auf der Basis von Polymethacrylaten. Man geht dabei von der sterilen feinpulvrigen Polymerphase aus, die neben den Hauptbestandteilen PMMA und PHEMA weitere Zusätze wie Initiatoren, Röntgekontrastmittel, Farbstoffe, Antibiotika u. dgl. in abgestimmten Mengen enthalten können. Für die Verarbeitung als Knochenzement sind selbstverständlich sterile Bedingungen einzuhalten. Die Monomerphase besteht aus flüssigem MMA und/oder HEMA, der weitere Zusätze wie Aktivatoren, Inhibitoren u. dgl. zugefügt sein können. Polymer- und Monomerphase werden in bestimmten Dosierungen miteinander vermischt, wobei man zweckmäßig das Pulver portionsweise in kurzer Zeit in die Flüssigkeit einträgt und kräftig verrührt. Man erhält einen knetbaren, plastisch verformbaren Teig, der nach ca. 2 Minuten auszuhärten beginnt. Die Aushärtung (und damit die Polymerisation) ist im allgemeinen nach etwa 10 Minuten beendet. Der Knochenzement bleibt etwa 6 Minuten plastisch, so daß er, beispielsweise zur Einzementierung von Endoprothesen, genügend lange gehandhabt werden kann. Zweckmäßig erfolgt die Eintragung des plastischen Materials erst kurz vor dem vollständigen Aushärten, da dann eventuelle nachteilige Wirkungen der Monomeren auf das Befinden des Patienten, z.B. Blutdruckabfall, Verlangsamung der Herz- und Atemfrequenz u.a., mit Sicherheit vermieden werden.

Dementsprechend ist das erfindungsgemäße Verfahren zur Herstellung einer selbshärtenden Masse auf der Basis von Polymethacrylaten aus einem Gemisch aus einer feinpulvrigen, festen Polymerphase aus Polymethylmethacrylat und einer flüssigen Monomerphase aus Methylmethacrylat, ggf. zusammen mit 2-Hydroxyethylmethacrylat, wobei man weitere übliche Additive, wie geeignete Initiatoren, Aktivatoren, Röntenkontrastmittel, Inhibitoren und dergleichen, einer oder beiden Phasen zumischt und das erhaltene Gemisch bei Raumtemperatur kräftig rührt und bei dieser Temperatur hält und wobei ein knetbarer, plastisch verformbarer Teig erhalten wird, der nach etwa 2 Minuten auszuhärten beginnt und nach etwa 10 Minuten ausgehärtet ist, dadurch gekennzeichnet, daß eine feste Polymerphase eingesetzt wird, die feinpulvriges Poly-2-hydroxyethylmethacrylat enthält, wobei das Gewichtsverhältnis von Polymerphase zu Monomerphase auf 1,5 bis 3,3:1 und der Anteil an Poly-2-hydroxyethylmethacrylat in dem ausgehärteten Produkt auf mindestes 2 Gew.% und höchstens 99 Gew.% eingestellt wird.

Vorzugsweise werden Polymerphase und Monomerphase im Gewichtsverhältnis von 2,0 bis 3,0:1 eingesetzt. Die Polymerphase wird zweckmäßig in feinpulvriger Form verwendet, wobei eine durchschnittliche Korngröße von 0,1 µm bis 0,5 mm bevorzugt eingesetzt wird.

In einer besonderen Ausgestaltung der Erfindung führt man das Verfahren mit einem Gemisch von PMMA und PHEMA im Gewichtsverhältnis von 3,3 bis 10:1 als Polymerphase und mit MMA als Monomerphase durch.

Beispiel 1 (Vergleichsbeispiel)

Eine als Knochenzement bekannte, selbsthärtende Masse wurde wie folgt hergestellt:

40 g Polymethylmethacrylat (PMMA) mit etwa 15 Gew.% Zirkondioxid (sog. "Palacos R" der Fa. Kulzer & Co. GmbH, Bad Homburg) und etwa 0,5 Gew.% Dibenzoylperoxid als Katalysator bildeten die Festphase und wurden zu

20 g Methylmethacrylat (MMA) (Dichte: 0,9433 bei 20°C) mit 0,7 Gew.% N,N-Dimethyl-p-toluidin als Cokatalysator und 0,006 Gew.% Hydrochinon und Chlorophyll als Flüssigphase zugefügt und

die Komponenten unter kräftigem Rühren bei Raumtemperatur (24°C) vermischt, wobei man unter Wärmeentwicklung eine plastische, formbare Masse erhielt.

Diese plastische Masse wurde bei einer Raum- und Materialtemperatur von 24°C als Knochenzement zum Einfüllen in die ausgeräumte Markhöhle eines Knochens verarbeitet, sobald sich ein knetbarer Teig gebildet hatte. Dies war etwa eine Minute nach Mischungsbeginn der Fall, wobei die Masse allerdings noch schmierig und nicht frei von Klebrigkeit war. Die Verarbeitbarkeit endete nach etwa 3 bis 4 Minuten, da zu diesem Zeitpunkt die Masse gummi-elastisch wurde.

Beispiel 2

Eine selbsthärtende Masse gemäß der Erfindung wurde wie folgt hergestellt:

Eine Festphase aus

33,80 g eines perlförmigen, im Handel befindlichen Polymethylmethacrylats mit einer Korngröße im Bereich von 0,01 bis 0,1 mm

6,00 g Zirkondioxid als Röntgenkontrastmittel

10,00 g Poly-2-hydroxyethylmethacrylat (PHEMA) mit einer Korngröße im Bereich von 0,1 bis 0,5 mm und

0,2 g Dibenzoylperoxid als Katalysator

wurden in eine Flüssigphase aus

20,00 g Methylmethacrylat mit 0,5 Gew.% N,N-Dimethyl-p-toluidin und 0,005 Gew.% Hydrochinon eingetragen und entsprechend, wie in Beispiel 1 angegeben, verarbeitet.

Die Verarbeitung als Knochenzement war bei einer Raum- und Materialtemperatur von 24°C ebenfalls 1 Minute nach Mischungsbeginn möglich, wobei die Masse weder schmierig noch klebrig war, sondern sich vielmehr ohne Rest leicht von der Unterlage, den Instrumenten und Gummihandschuhen ablösen ließ. Die Verarbeitbarkeit war auch 6 Minuten später noch ohne weiteres möglich und endete nach etwa 7 bis 8 Minuten.

Beispiele 3 bis 5

In der gleichen Weise, wie in Beispiel 1 beschrieben, wurden erfindungsgemäß selbsthärtende Massen unter Zugrundelegung der in Beispiel 2 angegebenen Zusammensetzung der Fest- und Flüssigphase hergestellt, mit dem Unterschied, daß die Festphase wechselnde Mengen Poly-2-hydroxyethylmethacrylat (PHEMA) enthielt, nämlich im 3. Biespiel 3,50 g PHEMA, im 4. Biespiel 5,50 g PHEMA und im 5. Biespiel 7,50 g PHEMA. In allen Fällen wurde eine Masse erhalten, die sich in der gleichen Weise und im gleichen Verarbeitungszeitraum, wie in Beispiel 2 angegeben, als Knochenzement verarbeiten ließ.

Die nachfolgenden Untersuchungen zur Bestimmung der mechanischen Eigenschaften von selbsthärtenden Massen verschiedener Zusammensetzung wurden nach Möglichkeit unter körperähnlichen Umgebungsbedingungen durchgeführt. Die Probenherstellung, die Probeformen und Probeabmessungen entsprechen den Vorschriften, die in der Monographie von O., K. Müller und W. Hupfauer, "Die Knochenzemente", Stuttgart 1975, Seiten 110 ff., beschrieben sind. Dementsprechend wurden zur Bestimmung der Reißfestigkeit nach DIN 53 455 und Reißdehnung (ebenfalls nach DIN 53 455) als Probekörper sog. "Schulterstäbe" verwendet, d.h. prismatische Stäbe mit paralleler Meßlänge und Verbreiterungen an beiden Enden in Form einer "Schulter", die in Probeformen Nr. 3 nach DIN 53 455 hergestellt wurden und deren mittlerer, zu prüfender Teil parallele Meßlängen der Abmes-

sungen 60 mm×10 mm×3 bis 4 mm aufwies. Zur Versuchsanordnung siehe z.B. Oest, et al, "Knochenzemente", Stuttgart 1975, Seite 147, Abb. 8.41 und 8.42.

Die Quetschspannung, d.h das Verhalten der Knochenzemente bei Druckbeanspruchung, wurde nach DIN 53 454 mit zylindrischen Probekörpern, die 12,5 mm lang und 10 mm im Durchmesser waren, bestimmt. Die Versuchsanordnung wurde z.B. in der oben zitierten Monographie "Knochenzemente", Seite 155/156, Abb. 8.51 und 8.52 beschrieben.

Die Bestimmung der Haftkraft erfolgte mit Hilfe von Durchdrückversuchen, bei denen mit Knochenzementmasse gefüllte zylindrische Keramikrohre als Probekörper eingesetzt wurden. Die ausführliche Beschreibung der Versuchsanordnung ist weiter unten vor Tabelle II angegeben.

Für die Messung des Elastizitätsmoduls nach DIN 53 457 wurden Normkleinstäbe verwendet, die in Probenform 2 nach DIN 53 452 mit den Abmessungen 50 mm×6 mm×4 mm hergestellt wurden.

Sämtliche Messungen wurden mit einer Zugprüfmaschine (Typ 1547, Hersteller Fa. K. Frank. Weinheim) durchgeführt.

Die in der folgenden Tabelle I zusammengestellten Ergebnisse stellen Mittelwerte auf jeweils 10 Messungen dar.

TABELLE I:
Mechanische Eigenschaften von a) einem bekannten PMMA-Knochenzement (Palacos) und b) der
erfindungsgemäßen Masse, hergestellt gemäß Beispiel 2

| Versuch Nr. | gemessene mech. Eigenschaft | (Dimension) | bekannter PMMA-Knochenzement (gemäß Beispiel 1) | | PMMA/PHEMA/MMA-Zement (gemäß Beispiel 2) | |
|---|---|---|---|---|---|---|
| | | | ungequollen[x] | gequollen[xx] | ungeguollen[x] | gequollen[xx] |
| 1 | Reißfestigkeit | $(N\,mm^{-2})$ | $44,3\pm4,0$ | $44,4\pm1,6$ | $34,5\pm1,5$ | $34,4\pm2,2$ |
| 2 | Reißdehnung | (%) | $3,3\pm0,4$ | $3,6\pm0,3$ | $2,5\pm0,2$ | $3,6\pm0,4$ |
| 3 | Quetschspannung | $(N\,mm^{-2})$ | $99,9\pm3,2$ | $97,8\pm6.0$ | $93,6\pm3,6$ | $87,2\pm4,1$ |
| 4 | Haftkraft | (N) | $250\pm390$ | $370\pm240$ | $750\pm650$ | $1650\pm460$ |
| 5 | Elastizitätsmodul | $(N\,mm^{-2})$ | $1644\pm70$ | $1590\pm140$ | $1650\pm120$ | $1170\pm100$ |

[x]gemessen nach eintägiger Lagerung bei 20°C und 65% r.F.
[xx]gemessen nach eintägiger Quellung bei 20°C in Ringerlösung

Die Ergebnisse in Tabelle I zeigen sehr deutlich, daß sich die gemessenen mechanischen Eigenschaften der erfindungsgemäßen Masse von denen des bekannten Knochenzements bis auf die Haftkraft nicht wesentlich unterscheiden. In der Haftkraft ist der Unterschied sowohl im gequollenen als auch im ungequollenen Zustand dagegen außergewöhnlich groß; die erfindungsgemäße Masse Haftet im ungequollenen Zustand etwa dreimal, im gequollenen Zustand sogar etwa viermal stärker als der bekannte Knochenzement.

In der nachfolgenden Tabelle II sind die Ergebnisse von Haftfestigkeitsmessungen zusammengestellt, die mit Hilfe von Durchdrückversuchen ermittelt wurden. Hierzu wurden 25 mm lange poröse Keramikrohre (Degussa) mit einem Außendurchmesser von 26 mm und einem Innendurchmesser von 18 mm mit der zu prüfenden Knochenzementmasse gefüllt und bei 20°C an der Luft bei 65% r.F. sowie eine Parallelprobe gleichzeitig bei 20°C in Ringerlösung gelagert. Dabei wurden zwei Versuchsreihen durchgeführt, eine mit einer Lagerungszeit von einem Tag, die zweite mit einer solchen von 28 Tagen. Anschließend wurden die Proben in einer Zugfestigkeitsprüfmaschine (Hersteller: K. Frank GmbH, Mannheim) mit einer Zuggeschwindigkeit von 10 mm/min geprüft. Dabei wurde die Zementfüllung mit Hilfe eines Metallstempels aus dem Keramikrohr gedrückt. Die Maximalkraft, die zum durchdrücken der Zementfüllung erforderlich war, wurde gemessen und diente als Maß für die Haftfestigkeit der untersuchten Knochenzementmassen an der Keramikfläche.

TABELLE II:

Haftfestigkeit von Knochenzementen an einer porösen Keramikwand

| Knochenzementproben | | Lagerungs-zeit (Tage) | Lagerungs-bedingungen bei 20°C | Haftfestigkeit (N) |
|---|---|---|---|---|
| aus Beispiel | Zusammensetzung | | | |
| 1 | PMMA/MMA (bekannte Masse) | 1 | 65 r.F. | 1290±350 |
| 2 | PMMA/PHEMA/MMA | 1 | 65 r.F. | 1380±590 |
| 5 | PMMA/PHEMA/MMA | 1 | 65 r.F. | 1570±750 |
| 1 | PMMA/MMA (bekannte Masse) | 28 | 65 r.F. | 1080±447 |
| 2 | PMMA/PHEMA/MMA | 28 | 65 r.F. | 1879±536 |
| 1 | PMMA/MMA (bekannte Masse) | 1 | Ringerlösung | 158± 37 |
| 2 | PMMA/PHEMA/MMA | 1 | Ringerlösung | 1070±187 |
| 5 | PMMA/PHEMA/MMA | 1 | Ringerlösung | 721±250 |
| 1 | PMMA/MMA (bekannte Masse) | 28 | Ringerlösung | 146± 53 |
| 2 | PMMA/PHEMA/MMA | 28 | Ringerlösung | 1216±574 |

Bei sämtlichen Werten handelt es sich um Mittelwerte aus je 10 Bestimmungen.

Aus der Tabelle II geht hervor, daß die erfindungsgemäßen Massen sowohl nach 1-tägiger als auch besonders ausgeprägt nach 28-tägiger Quellung in Ringerlösung bei 20°C eine wesentlich höhere Haftfestigkeit als der bekannte Knochenzement "Palacos" aufweisen.

Wie oben bereits ausführlich erläutert, zeichnet sich die erfindungsgemäße selbsthärtende Masse durch ein ausgeprägtes Quellvermögen aus, auf dem offenbar wenigstens zum Teil die Vorteile beruhen, die bei Verwendung dieser Masse als Knochenzement hinsichtlich Funktionstüchtigkeit und Lebensdauer von implantierten Prothesen erhalten werden.

Zur Bestimmung des Quellverhaltens wurden als Knochenzement-Probekörper Normkleinstäbe gemäß Probenform 2 nach DIN 53 452 mit einer Länge von 50 mm, einer Breite von 6 mm und einer Höhe von 4 mm sowohl aus einem bekannten Knochenzement aus PMMA/MMA (Palacos) als auch aus der erfindungsgemäßen Masse PMMA/PHEMA/MMA gemäß Beispiel 2 hergestellt. Die erhaltenen Normkleinstäbe wurden bei 20°C so in Ringer'sche Lösung gelegt, daß sie von ihr vollkommen bedeckt waren. Nach bestimmten Zeitabschnitten wurde die jeweilige Gewichtsänderung der Probekörper bestimmt. Die Ergebnisse sind in Tabelle III zusammengestellt und lassen erkennen, daß die Gewichtszunahme (durch Wasseraufnahme) in Abhängigkeit von der Quellzeit ein Charakteristikum für das Quellverhalten des betreffenden Knochenzements ist. Danach ist das Quellvermögen der erfindungsgemäßen Masse etwa 2,5 mal größer als das des untersuchten bekannten Knochenzements.

**0 009 754**

TABELLE III:

Quellvermögen von Knochenzement-Normkleinstäben in Ringerlösung bei 20°C

| Quellzeit (in Wochen) | Gewichtszunahme (in %, bezogen auf das Anfangsgewicht) | |
|---|---|---|
| | bekannte Masse PMMA/MMA (n, Beispiel 1) | erfindungsgem. Masse PMMA/PHEMA/MMA (n. Beispiel 2) |
| 0,5 | 0,8 | 1,6 |
| 1 | 1,3 | 2,6 |
| 2 | 1,3 | 3,0 |
| 3 | 1,4 | 3,3 |
| 4 | 1,4 | 3,7 |
| 5 | 1,5 | 4,1 |
| 6 | 1,6 | 4,4 |
| 7 | 1,7 | 4,7 |
| 8 | 1,7 | 4,7 |
| 9 | 1,7 | 4,8 |
| 10 | 1,7 | 4,9 |
| 11 | 1,7 | 4,9 |
| 12 | 1,8 | 5,0 |

Ein quellfähiges Material dehnt sich bekanntlich beim Quellen aus und übt dabei einen gewissen Quelldruck auf seine Umgebung aus, sofern es an der Ausdehnung gehindert wird. Da, wie oben beschrieben, die Ausbildung eines Quelldruckes durch einen in Gegenwart von Körperflüssigkeit quellenden Knochenzement eine Verstärkung des Knochens durch Wachstumsstimulation bewirken kann, wodurch der Knochen gekräftigt und gegen mechanische Belastungen widerstandsfähiger gemacht wird, was wiederum die Funktionsfähigkeit und Lebensdauer der Prothesen positiv beeinflußt, ist die Kenntnis des Quelldruckes wichtig füg die Beurteilung der Qualität von Knochenzementen.

Zur Bestimmung des Quelldruckes von Knochenzement wurden zylindrische Probekörper von 30 mm Länge und 24 mm Durchmesser in eine perforierte Metallhülse eingelegt und mittels Schraubverschlüssen an den Enden der Metallhülse gehalten. In einen der beiden Schraubverschlüsse war eine Druckmeßdose mit einem Meßbereich von 0 bis 50 bar eingebaut. Mit dem zweiten Schraubverschluß, der ein Feingewinde aufwies, wurde bei Versuchsbeginn ein Vordruck von ca. 1 bar eingestellt. Die so vorbereiteten Proben wurden in Ringerlösung von 20°C eingelegt, so daß sie von der Lösung vollständig bedeckt waren, und eine bestimmte Zeit lang in der Lösung gelagert. Der sich durch Quellung des Probekörpers aufbauende Druck wurde laufend registriert.

Als Probekörper wurden ein bekannter Knochenzement gemäß Beispiel 1 (Palacos) und eine erfindungsgemäß zusammengesetzte selbsthärtende Masse gemäß Beispiel 2 (PMMA/PHEMA/MMA) untersucht. Die Ergebnisse sind in den Tabellen IVa und IVb zusammengestellt.

TABELLE IVa:

Quelldruck von Knochenzementen bei 20°C

| Quellzeit (Stdn.) | Quelldruck (in bar) von | |
|---|---|---|
| | PMMA/MMA (n. Beispiel 1) | PMMA/PHEMA/MMA (n. Beispiel 2) |
| 0 | 1,0 (Vordruck) | 1,0 (Vordruck) |
| 2 | 1,0 | 1,6 |
| 4 | 1,1 | 2,1 |
| 6 | 1,2 | 2,4 |
| 8 | 1,25 | 2,5 |
| 10 | 1,25 | 2,5 |
| 12 | 1,25 | 2,6 |
| 14 | 1,25 | 2,65 |
| 16 | 1,25 | 2,75 |
| 18 | 1,25 | 2,8 |
| 20 | 1,25 | 2,85 |
| 22 | 1,3 | 2,9 |
| 24 | 1,3 | 3,0 |

9

TABELLE IVb:
Quelldruck von PMMA/PHEMA/MMA (n. Beispiel 2) (Langzeitversuch)

| Quellzeit (Wochen) | Quelldruck (in bar) | Quellzeit (Wochen) | Quelldruck (in bar) |
|---|---|---|---|
| 0 | 1 (Vordruck) | 8 | 19,5 |
| 1 | 4,8 | 9 | 21,0 |
| 2 | 8,5 | 10 | 22,5 |
| 3 | 11,0 | 11 | 24,0 |
| 4 | 12,5 | 12 | 25,0 |
| 5 | 14,5 | 13 | 26,5 |
| 6 | 16,5 | 14 | 27,5 |
| 7 | 18,0 | 15 | 28,5 |

Die in den Tabellen IVa und IVb angegebenen Meßergebnisse sind Mittelwerte aus jeweils 10 Versuchen.

Die Ergebnisse in Tabelle IVa zeigen sehr deutlich den wesentlich stärkeren Quelldruck der erfindungsgemäßen Masse, der bereits nach 4 Stdn. etwa doppelt so groß wie der vorher eingestellt Vordruck ist, während in demselben Zeitraum die bekannte Knochenzementmasse keinen nennenswerten Quelldruck aufgebaut hat. Nach 24 Stdn. hat sich der Quelldruck der erfindungsgemäßen Masse bereits verdreifacht, während er für den bekannten Knochenzement erst 1/3 des Vordruckes beträgt.

Tabelle IVb zeigt, daß der Quelldruck der erfindungsgemäßen Masse auch über viele Wochen ständig weiter zunimmt und dabei Werte erreicht, die ausreichen, um bei Verwendung als Knochenzement eine merkliche Wirkung auf den umgebenden Knochen auszuüben.

**Patentansprüche**

1. Selbsthärtende Masse auf der Basis von Polymethacrylaten, hergestellt aus einem Gemisch aus einer feinpulvrigen, festen Polymerphase aus Polymethylmethacrylat und einer flüssigen Monomerphase aus Methylmethacrylat, ggf. zusammen mit 2-Hydroxyethylmethacrylat, unter Zumischen weiterer üblicher Additive zu einer oder beiden Phasen und kräftigem Rühren des erhaltenen Gemisches bei Raumtemperatur, wobei man einen knetbaren, plastisch verformbaren Teig erhält, der nach etwa 2 Minuten auszuhärten beginnt und nach etwa 10 Minuten ausgehärtet ist, dadurch gekennzeichnet, daß die feste Polymerphase feinpulvriges Poly-2-hydroxyethylmethacrylat enthält, wobei das Gewichtsverhältnis von Polymerphase zu Monomerphase im Bereich von 1,5 bis 3,3:1 liegt und der Anteil an Poly-2-hydroxyethylmethacrylat in dem ausgehärteten Produkt mindestens 2 Gew.% und höchstens 99 Gew.% beträgt.

2. Masse nach Anspruch 1, dadurch gekennzeichnet, daß das Gewichtsverhältnis von Polymerphase zu Monomerphase im Bereich von 2,0 bis 3,0:1 liegt.

3. Masse nach den Ansprüchen 1 oder 2, dadurch gekennzeichnet, daß die Polymerphase in einer durchschnittlichen Korngröße von 0,1 $\mu$m bis 0,5 mm vorliegt.

4. Masse nach den Ansprüchen 1 bis 3, dadurch gekennzeichnet, daß die Polymerphase Dibenzoylperoxid als Initiator und die Monomerphase N,N-Dimethyl-p-toluidin als Aktivator enthält.

5. Masse nach den Ansprüchen 1 bis 4, dadurch gekennzeichnet, daß die Polymerphase aus einem Gemisch von Polymethylmethacrylat und Poly-2-hydroxyethylmethacrylat im Gewichtsverhältnis von 3,3 bis 10:1 und die Monomerphase aus Methylmethacrylat besteht.

6. Verfahren zur Herstellung einer selbsthärtenden Masse auf der Basis von Polymethacrylaten aus einem Gemisch aus einer feinpulvrigen, festen Polymerphase aus Polymethylmethacrylat und einer flüssigen Monomerphase aus Methylmethacrylat, ggf. zusammen mit 2-Hydroxyethylmethacrylat, wobei man weitere übliche Additive einer oder beiden Phasen zumischt und das erhaltene Gemisch bei Raumtemperatur kräftig rührt, wobei ein knetbarer, plastisch verformbarer Teig erhalten wird, der nach etwa 2 Minuten auszuhärten beginnt und nach etwa 10 Minuten ausgehärtet ist, dadurch gekennzeichnet, daß eine feste Polymerphase eingesetzt wird, die feinpulvriges Poly-2-hydroxyethylmethacrylat enthält, wobei das Gewichtsverhältnis von Polymerphase zu Monomerphase auf 1,5 bis 3,3:1 und der Anteil an Poly-2-hydroxyethylmethacrylat in dem ausgehärteten Produkt auf mindestens 2 Gew.% und höchstens 99 Gew.% eingestellt wird.

7. Verfahren nach Anspruch 6, dadurch gekennzeichnet, daß man als Initiator Dibenzoylperoxid und als Aktivator N,N-Dimethyl-p-toluidin einsetzt.

8. Verfahren nach den Ansprüchen 6 und 7, dadurch gekennzeichnet, daß man als Polymerphase ein Gemisch von Polymethylmethacrylat und Poly-2-hydroxyethylmethacrylat im Gewichtsverhältnis von 3,3 bis 10:1 und als Monomerphase Methylmethacrylat einsetzt.

9. Verwendung der selbsthärtenden Massen nach den Ansprüchen 1 bis 5 als Knochenzement in der Chirurgie und Orthopädie.

## 0 009 754

**Claims**

1. A self-curing mass based on polymethacrylates prepared from a mixture of a finely powdered solid polymer phase comprising polymethyl methacrylate and a liquid monomer phase comprising monomeric methyl methacrylate, optionally together with monomeric 2-hydroxyethyl methacrylate, admixing further conventional additives into one or both phases and vigorously stirring the resulting mixture at room temperature thereby obtaining a kneadable, plastic shapable paste which begins to cure after about two minutes and which is cured after about 10 minutes, characterized in that the solid polymer phase comprises a finely powdered poly-(2-hydroxyethyl methacrylate), whereby the weight ratio of polymer phase to monomer phase is in the range of 1.5 to 3.3:1 and wherein the portion of poly-(2-hydroxyethyl methacrylate) in the cured product is at least 2% by weight and at most 99% by weight.

2. A mass according to claim 1, characterized in that the weight ratio of polymer phase to monomer phase is in the range of 2.0 to 3.0:1.

3. A mass according to claim 1 or 2, characterized in that the average particle size of the polymer phase is from 0.1 $\mu$m to 0.5 mm.

4. A mass according to claims 1 to 3, characterized in that the polymer phase comprises dibenzoyl peroxide as an initiator and the monomer phase comprises N,N-dimethyl-p-toluidine as an activator.

5. A mass according to claims 1 to 4, characterized in that the polymer phase consists of polymethyl methacrylate and poly-(2-hydroxyethyl methacrylate) in the weight ratio of 3.3 to 10:1 and the monomer phase consists of methyl methacrylate.

6. A process of preparing a self-curing mass based on polymethacrylates from a mixture of a finely powdered solid polymer phase comprising polymethyl methacrylate and a liquid monomer phase comprising monomeric methyl methacrylate, optionally together with monomeric 2-hydroxyethyl methacrylate, admixing further conventional additives into one or both phases and vigorously stirring the mixture at room temperature thereby obtaining a kneadable, plastic shapable paste which begins to cure after about 2 minutes and which is cured after about 10 minutes, characterized in that a solid polymer phase is used which comprises a finely powdered poly-(2-hydroxyethyl methacrylate), the weight ratio of polymer phase to monomer phase being in the range of 1.5 to 3.3:1 and the portion of poly-(2-hydroxyethyl methacrylate) in the cured product being in the range of at least 2% by weight and at most 99% by weight.

7. A process according to claim 6, wherein dibenzoyl peroxide is used as an initiator and N,N-dimethyl-p-toluidine is used as an activator.

8. A process according to claims 6 and 7, wherein the polymer phase consists of a mixture of polymethyl methacrylate and poly-(2-hydroxyethyl methacrylate) in the weight ratio of 3.3 to 10:1 and the monomer phase consists of methyl methacrylate.

9. Use of the self-curing mass according to claims 1 to 5 as bone cement in the surgery and orthopaedy.

**Revendications**

1. Matière auto-durcissante à base de polyméthacrylates, préparée à partir d'un mélange d'une phase polymère de poly(méthacrylate de méthyle) solide en fines particules et d'une phase monomère liquide constituée de méthacrylate de méthyle et éventuellement de méthacrylate de 2-hydroxy-éthyle, d'autres additifs usuels étant ajoutés à l'une des deux phases ou aux deux et le mélange étant soumis à une agitation vigoureuse à la température ordinaire jusqu'à l'obtention d'une pâte malléable, plastiquement déformable, dont la prise débute après environ deux minutes et est complète après environ dix minutes, caractérisée en ce que la phase polymère solide contient du poly(méthacrylate de 2-hydroxy-éthyle) en fines particules, les proportions pondérales de la phase polymère et de la phase monomère sont dans un rapport compris entre 1,5:1 et 3,3:1 et la proportion du poly(méthacrylate de 2-hydroxy-éthyle) dans le produit durci est de 2% en poids au moins et de 99% en poids au plus.

2. Matière suivant la revendication 1, caractérisée en ce que le rapport pondéral de la phase polymère à la phase monomère est compris entre 2,0:1 et 3,0:1.

3. Matière suivant les revendications 1 et 2, caractérisée en ce que la phase polymère possède une granulométrie moyenne comprise entre 0,1 $\mu$m et 0,5 mm.

4. Matière suivant les revendications 1 à 3, caractérisée en ce que la phase polymère contient en tant qu'initiateur du peroxyde de dibenzoyle et la phase monomère en tant qu'activateur de la N,N'-diméthyl-p-toluidine.

5. Matière suivant les revendications 1 à 4, caractérisée en ce que la phase polymère est composée d'un mélange de poly(méthacrylate de méthyle) et de poly(méthacrylate de 2-hydroxy-éthyle) dans un rapport pondéral de 3,3:1 à 10:1 et la phase monomère est formée de méthacrylate de méthyle.

6. Procédé de préparation d'une matière autodurcissante à base de polyméthacrylates à partir d'un mélange d'une phase polymère de poly(méthacrylate de méthyle) solide en fines particules et d'une phase monomère liquide constituée de méthacrylate de méthyle et éventuellement de méthacrylate de 2-hydroxy-éthyle, d'autres additifs usuels étant incorporés à l'une des phases ou aux deux et le mélange étant soumis à un malaxage vigoureux à la température ordinaire jusqu'à l'obtention d'une pâte malléable, plastiquement déformable, dont la prise débute après environ deux minutes et est complète après environ dix minutes, caractérisé en ce que l'on utilise une phase polymère solide, contenant du poly(méthacrylate et 2-hydroxy-éthyle) en fines particules, le rapport pondéral entre la phase polymère et la phase monomère étant compris entre 1,5:1 et 3,3:1 et la teneur en poly(méthacrylate de 2-hydroxy-éthyle) du produit durci se situant entre 2% en poids au moins et 99% en poids au plus.

7. Procédé suivant la revendication 6, caractérisée en ce que l'initiateur est le peroxyde de dibenzoyle et l'activateur la N,N'-diméthyl-p-toluidine.

8. Procédé suivant les revendications 6 et 7, caractérisé en ce que la phase polymère est constituée d'un mélange de poly(méthacrylate de méthyle) et de poly(méthacrylate de 2-hydroxy-éthyle) dans un rapport pondéral de 3,3:1 à 10:1 et la phase monomère est formée de méthacrylate de méthyle.

9. Utilisation de matières auto-durcissantes suivant les revendications 1 à 5 comme ciment pour les os dans les domaines chirurgical et orthopédique.